# EUROPÄISCHE PATENTSCHRIFT

(11) **EP 1 476 223 B1**
(45) Veröffentlichungstag und Bekanntmachung des Hinweises auf die Patenterteilung: **13.02.2008**
(21) Anmeldenummer: 03742465.2
(22) Anmeldetag: 19.02.2003
(51) Int. Cl.: A61N 1/36, G01N 27/414, H01L 29/78

(54) **DOPPELGATE-TRANSISTOR-ANORDNUNG ZUR AUFNAHME VON ELEKTRISCHEN SIGNALEN VON LEBENDEN ZELLEN**
DOUBLE GATE TRANSISTOR ARRANGEMENT FOR RECEIVING ELECTRICAL SIGNALS FROM LIVING CELLS
SYSTEME DE TRANSISTOR A DOUBLE PORTE POUR RECEVOIR DES SIGNAUX ELECTRIQUES ISSUS DE CELLULES VIVANTES

(30) Priorität: 19.02.2002 AT 2522002
(43) Veröffentlichungstag der Anmeldung: 17.11.2004
(73) Patentinhaber: Austria Wirtschaftsservice Gesellschaft mit beschränkter Haftung, 1030 Wien (AT); Bertagnolli, Emmerich, 1190 Wien (AT)
(72) Erfinder: BERTAGNOLLI, Emmerich, A-1190 Wien (AT)
(74) Vertreter: Margotti, Herwig Franz
(86) Internationale Anmeldenummer: PCT/AT2003/000056
(87) Internationale Veröffentlichungsnummer: WO 2003/070321

(56) Entgegenhaltungen:
- US-A- 4 921 591
- US-A- 5 801 428
- US-B1- 6 181 969

## Beschreibung

Die Erfindung betrifft eine Anordnung zur Kopplung einer lebenden Zelle, insbesondere Nervenzelle, an eine elektronische Schaltung zur Aufnahme von direkt oder indirekt elektrisch wirksamen Zellsignalen und/oder zur elektrischen Reizung der Zelle.

Im Folgenden wird eine Interaktion der Kopplungsanordnung mit einer Zelle, bei der ganz allgemeine, direkt oder indirekt elektrisch wirksame Zellsignale, insbesondere aber elektrische Signale von Nervenzellen, aufgenommen und weiterverarbeitet werden, als Passivmodus bezeichnet. Eine Interaktion der Kopplungsanordnung mit einer Zelle, die eine elektrische Reizung der Zelle bewirkt, wird als Aktivmodus bezeichnet.

Ohne Einschränkung der Allgemeinheit des erfinderischen Ansatzes ist die vorgestellte Erfindung auch in großtechnischem Maßstab umsetzbar, d. h. auf der Basis von bestehenden Integrationsprozessen der Mikro- und Nanoelektronik verwirklichbar, wobei zunächst nur solche Prozesse und Materialien verwendet werden, die einerseits mit einer modernen Fertigungsumgebung für Siliziumchips kompatibel sind, und andererseits biologisch nicht schädlich sind. In weiterer Ausgestaltung sind jedoch auch andere datenverarbeitungsfähige elektronische Strukturen, wie sie etwa organische Schaltkreise darstellen, denkbar und möglich.

Bisherige Verfahren benutzen zum einen optische Methoden basierend auf der Spannungsabhängigkeit von Farbstoffen (z. B. Fluoreszenz), oder extrazelluläre Metallelektroden. Zum anderen bedienen sie sich invasiver Methoden, z. B. direkt in die Zelle eingestochener Elektroden zur Detektion von intrazellulären Potentialen. Zur Klasse der invasiven Elektroden gehören auch die Siliziumnadel, auf der photolithographisch erzeugte Leiterbahnen mit Kontaktpunkten an der Spitze vorgesehen sind.

Die umgekehrte Funktion, die elektrische Reizung einer Zelle erfolgte bisher ebenfalls durch eingestochene Elektroden.

Neben den erwähnten technischen Lösungen zur Detektion und Reizung von singulären Zellen gibt es noch sogenannte Cuff-Elektroden. Hierbei handelt es sich um eine mit einem Drahtnetz bespannte Manschette, die um eine Nervenfaser (incl. Versorgungs- und Stützgewebe) herum gelegt wird. Mit Cuff-Elektroden lassen sich aber nur summarisch Potentiale abgreifen bzw. einprägen.

Da invasiver Zugriff auf die Zelle stets gleichbedeutend mit einer Verletzung oder unmittelbaren oder mittelbaren Zerstörung der Zelle ist, besteht die Forderung nach hoher räumlicher und zeitlicher Auflösung und elektrischer Empfindlichkeit bei nicht invasivem (bzw. nicht penetrierendem) Zugriff auf die Zelle. Diese Forderung wird jedoch von keiner der bestehenden Vorrichtungen oder Methoden erfüllt.

Bisherige nicht invasive Lösungen auf der Basis von MOS-Transistoren beruhten im Kern auf der Beeinflussung der Inversionsschicht im Kanal eines MOSFET entweder direkt durch die Auflage der Nervenzelle bzw. eines Axons auf das Gatedielektrikum, oder indirekt durch die Auflage dieses elektrowirksamen Zellteiles auf das Gate.

Im einen Fall erfolgt der Kontakt zwischen der Zelle und dem Transistor in der Weise, dass ein Teil der Zellmembran auf dem Transistor im Kanalbereich aufliegt bzw. in Kontakt gebracht wird. Dieser Bereich ist durch das Gatedielektrikum, meist ein natürliches Oxid oder ein künstlich gewachsenes Oxid, vom Substrat getrennt. Ladungen bzw. Ladungsänderungen der aufliegenden Zelle bzw. der aufliegenden Zellmembran beeinflussen die Kanalleitfähigkeit des Transistors. Die bisher mit diesen Verfahren erreichten Kopplungstärken liegen im Bereich von 10% Gate-zu-Zellmembran-Potentialänderung. Der MOS-Transistor, abgedeckt durch Oxid, ist dabei bis auf die lateral weit herausgeführten Anschlüsse völlig in Elekrolytflüssigkeit, angereichert mit Nährlösung, getaucht, was seiner Lebensdauer extrem abträglich ist (Metallionen, insabesondere aber Na und K Ionen wandern in das Gatedielektrikum ein und führen zu einer fortschreitenden Degradation). Daneben ergeben sich allein schon aufgrund nicht vollständiger Abdeckungen des Kanalbereiches teilausgebildete Kanäle mit entsprechend variabler bzw. schwacher Kopplung.

Daher liefert dieser Ansatz nur eine eingeschränkte Lösung für einige wenige singuläre Schnittstellen zwischen Gewebe und Elektronik. Aufgrund der fehlenden regulären Verdrahtungsmöglichkeit des Transistors erlaubt diese Lösung nur eine begrenzte Anzahl von Abtastungszyklen und nur unter schwierigen Randbedingungen eine Verstärkung und Verarbeitung der Signale am Entstehungsort.

Des weiteren ist mit diesem System eine direkte aktuatorische Reizung grundsätzlich ausgeschlossen.

Ein zweiter bekannter Ansatz beruht auf der Ankopplung eines Neurons an das Gate eines MOS auf Basis eines direkten, metallisch ausgeführten galvanischen Kontaktes zwischen Zellmembran und Gate des Transistors. Auch in diesem Fall beruht das Messprinzip auf der Beeinflussung des Kanalbereichs des MOSFET durch die Aufladung des Gates. Als Vorteil ist zu werten, dass das empfindliche Gateoxid nicht direkt der Nährlösung ausgesetzt werden muss und daher eine höhere Lebensdauer dieses Systems zu erwarten ist, siehe z. Bsp. US-A-5 801 428.

Das Ausleseprinzip beruht in beiden Fällen auf dem Einsatz von Differenzverstärkern - im allgemeinen vom CMOS Typ - zur Detektion der Änderung der Leitfähigkeit des Kontakttransistors im Vergleich zu einem nicht kontaktierten, regulären Transistor.

Für die Reizung der Zelle sind in beiden Fällen eigene Reizleitungen und entsprechende Anschlusskonfigurationen nötig, z. B. durch Herstellung von Ringkontakten um den Kontakttransistor.

Der vorliegenden Erfindung liegt die Aufgabe zugrunde, eine Lösung für die oben beschriebenen Nachteile des Standes der Technik zu bieten.

Um diese Aufgabe zu erfüllen wird eine Anordnung zur Kopplung einer lebenden Zelle, insbesondere Nervenzelle, an eine elektronische Schaltung zur Aufnahme von direkt oder indirekt elektrisch wirksamen Zellsignalen und/oder zur elektrischen Reizung der Zelle gemäß den Merkmalen des Anspruchs 1 bereitgestellt. Vorteilhafte Ausgestaltungen der Erfindung sind in den Unteransprüchen dargelegt.

Im Gegensatz zu allen bisher bekannten Lösungsansätzen ist bei der erfindungsgemäßen Kopplungsanordnung die rein passive Aufnahme direkt oder indirekt elektrisch wirksamer Zellsignale ebenso möglich wie eine direkte aktive elektrische Reizung und Beeinflussung (Aktivierung) der Zelle. Die wahlweise Einstellung des passiven oder des aktiven Betriebsmodus wird jeweils durch die äußere Beschaltung und die Wahl der angelegten Spannungen und Ströme vorgenommen. Die Adressierung und die Abfrage sind rein passiver Natur, d.h. sie verändern das Potential bzw. den elektrischen Zustand der Zelle nicht.

Die Erfindung kann insbesondere in folgenden Themenfeldern Anwendung finden:
➢ Untersuchungen direkt oder indirekt elektrisch wirksamer Aktivitäten von Zellen und Mechanismen der Pulsfortleitung von Aktionspotentialen entlang von Axonen (Nervenfasern).
➢ Signalprozessierung in Netzwerken von lebenden Neuronen.
➢ Örtlich und zeitlich aufgelöste, gleichzeitige Detektion der Signal/Reizreaktionen einer großen Zahl von Zellen bzw. Neuronen und der damit zusammenhängenden Untersuchung der elektrischen Verschaltung von Zellen bzw. Neuronen.
➢ Bau oder Assemblierung von Biosensoren, insbesondere von neuronalen Biosensor- bzw Sensor-/Aktorsysteme und Arrays.
➢ Realisierung von neuron-elektronischen Schaltungen.

Weitere mögliche Anwendungen der Erfindung betreffen die Konstruktion von sensorischen und sensomotorischen Aktuatoren und Rezeptoren für Prothesen und die Realisierung von elektronischen Substituten für Nervenfasern, insbesondere für geschädigte oder zertrennte Nervenfasern.

Daneben bietet die Erfindung auch die Möglichkeit, die Wirkung von chemischen bzw. physikalischen Reizen, insbesondere aber von Medikamenten und bioaktiven Medien auf die Funktionsweise und Funktionsfähigkeit von Zellen bzw. Geweben und Gewebeteilen zu untersuchen.

Die Grundidee der Erfindung beruht darauf, dass eine lebende Zelle mit einem Gate eines mit doppeltem Gate ausgebildeten Transistors in Kontakt gebracht wird, so dass eine Änderung der elektrischen Eigenschaften der Zelle selbst, oder aber eines Teiles der Zelle, das Verhalten des Transistors steuert, was über eine Auswahlschaltung elektrisch erfasst werden kann. Diese Schaltung macht es auch möglich, wahlweise elektrische Signale bzw. Reize an diese Zelle oder an diesen Teil der Zelle zu bringen.

Biologische Zellen sind auf Dauer nur lebensfähig, wenn sie in einem flüssigen Milieu, im allgemeinen in einer so genannten Nährlösung gehalten werden. Dem entsprechend soll der erfindungsgemäße Aufbau der Schaltanordnung eine Flüssigkeitslagerung der biologischen Zelle erlauben. Dies wird erfindungsgemäß durch das Vorsehen eines Nährlösungs-Behältnisses gewährleistet, in dessen Innenraum zumindest ein Kontaktelement ragt oder diesen zumindest teilweise bildet.

Um ein definiertes Messpotential sicherzustellen, ist in einer Fortbildung der Erfindung eine mit einer Referenzspannung verbundene elektrisch leitende Referenzelektrode vorgesehen, die in den Innenraum des Nährlösungs-Behältnisses ragt.

Hohe Lebensdauer der erfindungsgemäßen Kopplungsanordnung und geringste Beeinflussung der damit gekoppelten Zellen können erzielt werden, wenn das elektrisch leitende Kontaktelement ein Material mit geringer biologischer Wirkung umfasst, vorzugsweise ausgewählt aus hochschmelzenden Metallen, wie Platin, Iridium, Osmium, Wolfram oder Gold, oder Legierungen daraus; oder aus Halbleitersiliziden, wie Platinsilizid, Wolframsilizid, Titansilizid; oder aus einem dotierten monokristallinen oder polykristallinen Halbleiter, wie leitfähigem Polysilizium; oder aus leitfähigen Kunststoffen.

Ungeachtet der Einfachheit der schematisch dargestellten Ausführungsbeispiele ist die erfindungsgemäße Kopplungsanordnung jedoch weder auf Einzelzellen beschränkt, noch auf in Flüssigkeit gelagerte Zellen.

Die Anordnung ist vielmehr hervorragend dazu geeignet, ganze Zellhaufen, Zellverbände, oder sogar ganze funktionale Zelleinheiten oder Kombinationen derselben elektrisch anzukoppeln. In diesem Fall werden im Flüssigkeitsbehälter anstelle von Einzelkontakten Kontaktfelder angeordnet, die jeweils elektrisch in der erfindungsgemäßen Weise angeschlossen und verbunden werden.

In Bezug auf die Lagerung der Zellen, Zellhaufen, Zellfunktionsgruppen oder Kombinationen derselben in einer Flüssigkeit bzw. einer Nährlösung gilt auch, dass jedes wässerige Umfeld, im speziellen also auch Körper- oder Gewebsflüssigkeiten ausreichen, um eine elektrolytische Ankopplung zu erreichen.

In einer zweckmäßigen Ausgestaltung der Erfindung ist eine Vielzahl von Kopplungsanordnungen in Form einer Matrix mit Zeilen und Spalten angeordnet, wobei der Eingang eines jeweiligen elektronischen Schalters mit einer Spalten-Adressleitung verbunden ist und ein Steueranschluss des elektronischen Schalters mit einer Zeilen-Adressleitung verbunden ist.

Es kann weiters eine Adressierschaltung zur einzelnen oder gruppenweisen Beaufschlagung von Spalten-Adressleitungen mit Versorgungs- oder Signalspannungen und von Zeilen-Adressleitungen mit Steuerspannungen vorgesehen sein. Wenn die Matrix also einen Aufbau in Form von i Zeilen und j Spalten aufweist, ermöglicht die Funktion des i-j-ten Elementes eine eindeutige räumliche Zuordnung auf dem Zellenfeld, das der Matrix zugeordnet ist. Bringt man eine Anzahl von Zellen, zum Beispiel Nervenzellen, mit einer solchen Matrix in Kontakt, so kann man die Zellen einzeln kontaktieren und im Passivmodus bzw. im Aktivmodus Signale austauschen. Wegen der Besonderheit der aufnehmenden Schaltung, nämlich der Matrizenform, können die elektrischen Signale - wie oben beschrieben - örtlich zugeordnet werden. Damit ist es möglich, von einer mehr oder weniger großen Anzahl von Zellen, die zum Beispiel einen funktionalen Verbund darstellen, die einzelnen aktiven Zellen zu ermitteln und mit diesen in Kontakt zu treten. Dieser Kontakt kann entweder nur im Passivmodus, oder im Aktivmodus, oder wahlweise abwechselnd in beiden Moden erfolgen.

Liegt nun ein größeres Zellensemble auf einer oder mehrerer solcher matrizenförmiger Zellenfelder von elektronischen Einzelzellen, so kann durch Aktivierung der elektronische Abfrage (Passiv-/Aktiv-Modus) ermittelt werden, an welcher Stelle welche Art von Kontakt vorliegt, welche räumliche Konfiguration diese Kontakte einnehmen, und in welcher Weise eine dem jeweiligen Anwendungszwecke entsprechende Signalabfolge bzw. welcher Algorithmus anzuwenden ist.

Nicht aktive, oder nicht ausreichend aktive, oder nicht kontaktierte Zellen können dabei ebenso ermittelt werden und z. B. von einer weiteren interaktiven Bearbeitung (Passiv- und/oder Aktivmodus) ausgeschlossen werden, indem die Adressierschaltung Adressierauswertemittel zur Erkennung von dysfunktionalen Zellen und von Fehlkontakten zwischen Kontaktelement und Zelle aufweist, wobei bei einer solchen Erkennung gegebenenfalls die weitere Interaktion dieser Zellen bzw. Kontaktelemente selektiv unterbrechbar ist. Zur selektiven Unterbrechung der Interaktion von Zellen können Unterbrechungsmittel, wie elektronische Schalter, vorgesehen sein.

Die erfindungsgemäße Kopplungsanordnung erlaubt die Anordnung einer Vielzahl von Kopplungsanordnungen auf einem Chip, wobei der Chip vorzugsweise in Si-Planartechnologie hergestellt und gegebenenfalls mit anderen Technologien, wie Schaltungen für lokale Verstärkung, "on-chip-logic", oder "systems on chips (SoC)" integriert ist.

Die nach dem Stand der Technik bereits heute erzielbaren Packungsdichten erlauben das Design von sehr großen Arrays von Zellsensoren und Aktoren, wobei wahlweise an beliebigen Knotenpunkten des Arrays lokal elektronische Schaltungen untergebracht werden können. Dies kann bei geringen Signalstärken (z. B. ist der Hub der Zellmembranspannung bei Aktivierung einer Nervenzelle typisch < 60 mV) und bei den auftretenden Rauschquellen und Störpegeln erforderlich sein.

Mit Hilfe dieser Sensorarrays und geeigneter Adressierschaltungen lässt sich die Signalausbreitung und die Signalverarbeitung in biologischen Zellen, Zellhaufen und funktionalen Zellsystemen, insbesondere in Nervenzellgeweben, beobachten bzw. messtechnisch erfassen und gegebenenfalls lassen sich die so gewonnenen Daten auch weiterverarbeiten.

In einer weiteren Ausgestaltung der Erfindung ist das Nährlösungs-Behältnis auf dem Chip angeordnet. Dies erlaubt die Flüssigkeitslagerung von ganzen Zellhaufen, Zellverbänden oder sogar ganzer funktionaler Zelleinheiten oder Kombinationen derselben im Kontaktbereich der Kontaktelemente und ermöglicht die Verwendung des Chips in wässrigem Umfeld, im speziellen auch Körper- oder Gewebsflüssigkeiten, die eine elektrolytische Ankopplung ermöglichen. Bei dieser Ausgestaltung ist die oberste Schicht des Chips, die sogenannte Passivierung, so auszuführen, dass die Kontaktpunkte oder -flächen der Kontaktelemente an speziellen Durchstoßungspunkten angebracht werden und diese dann mit den Zellen in Kontakt gebracht werden.

Die erfindungsgemäße Kopplungsanordnung kann direkt in lebendes Gewebe oder in Öffnungen desselben eingebracht werden. Für den Kontakt mit der Gewebeflüssigkeit, dem Elektrolyten, ist erfindungsgemäß ein geeigneter, gegebenenfalls auch integrierter Kontakt vorzusehen.

Im Folgenden werden Ausführungsbeispiele der Erfindung unter Bezugnahme auf die Zeichnungen erläutert. Die Erfindung ist jedoch nicht auf diese Beispiele beschränkt.

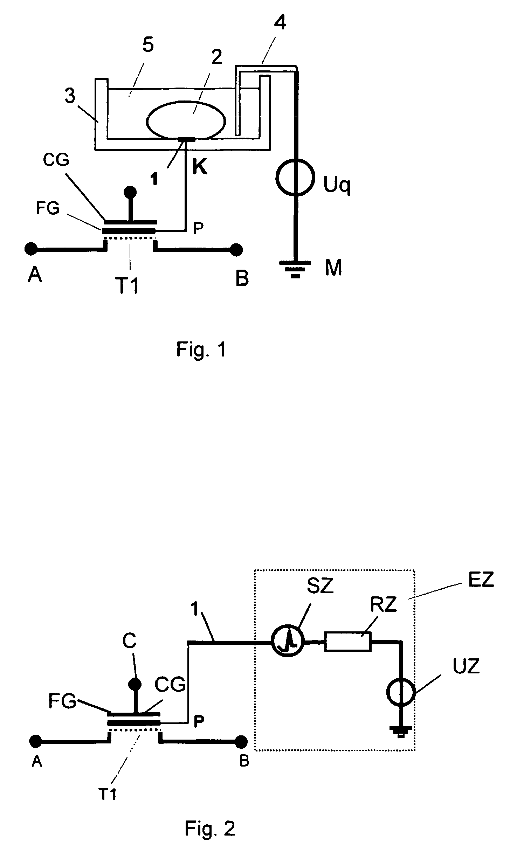

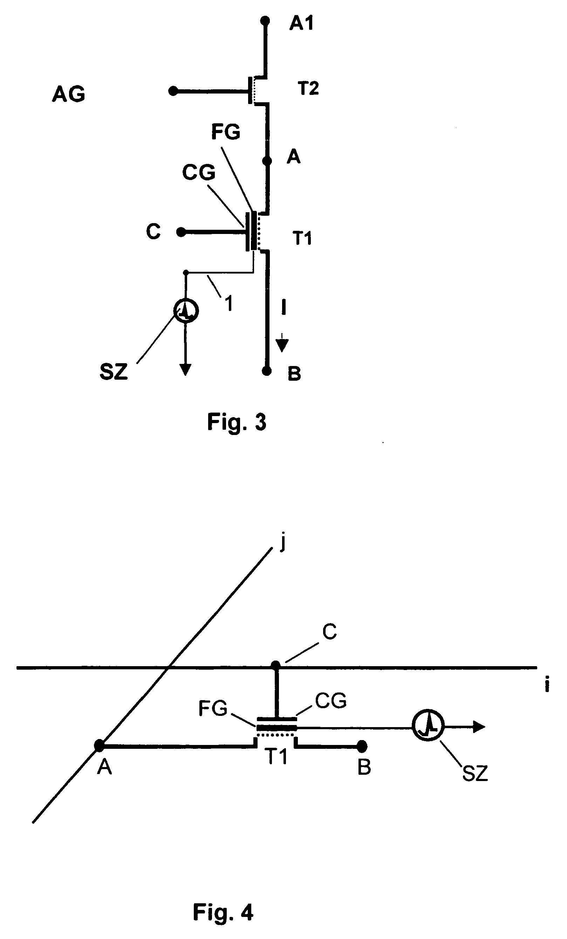

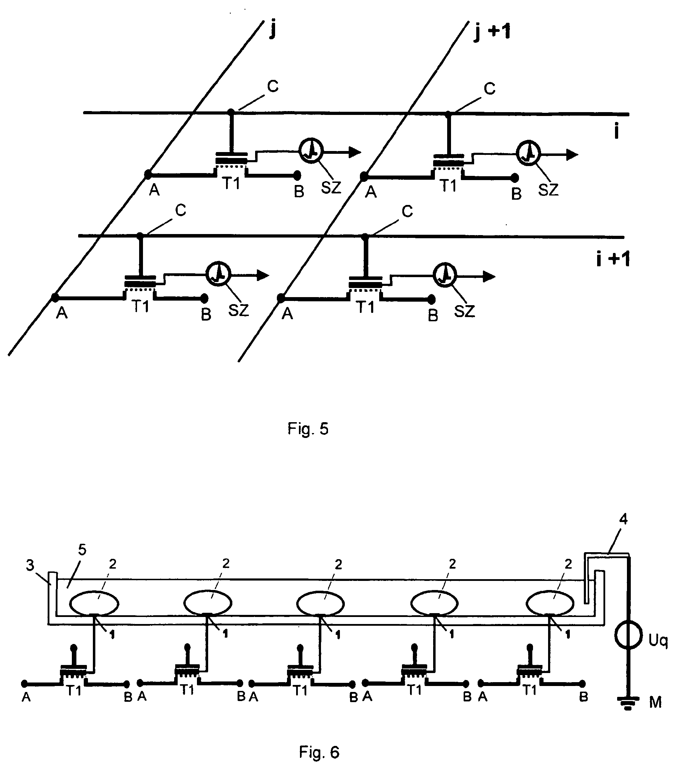

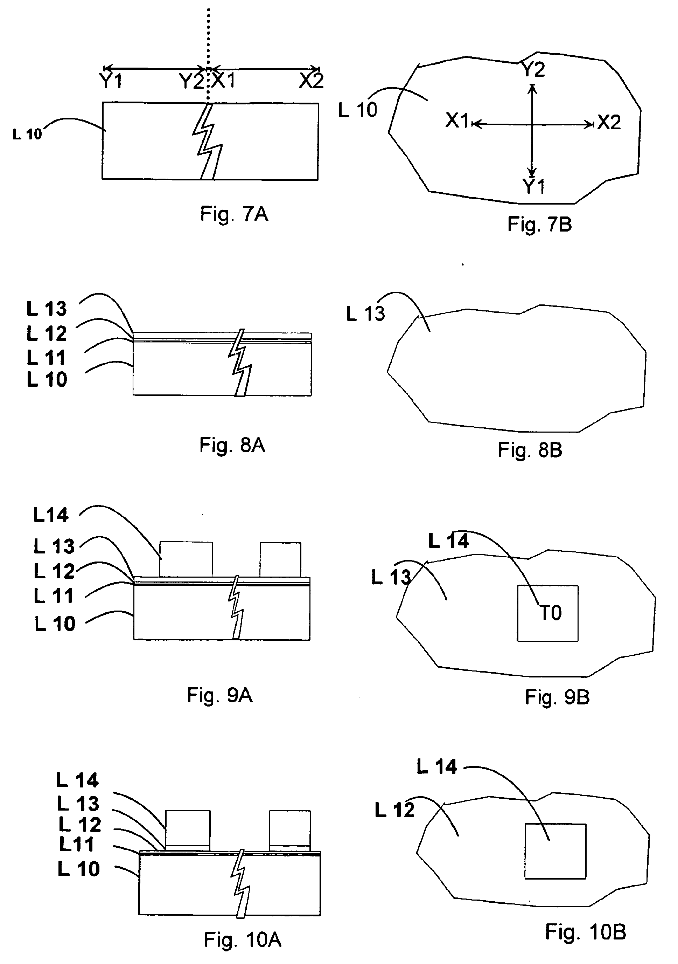

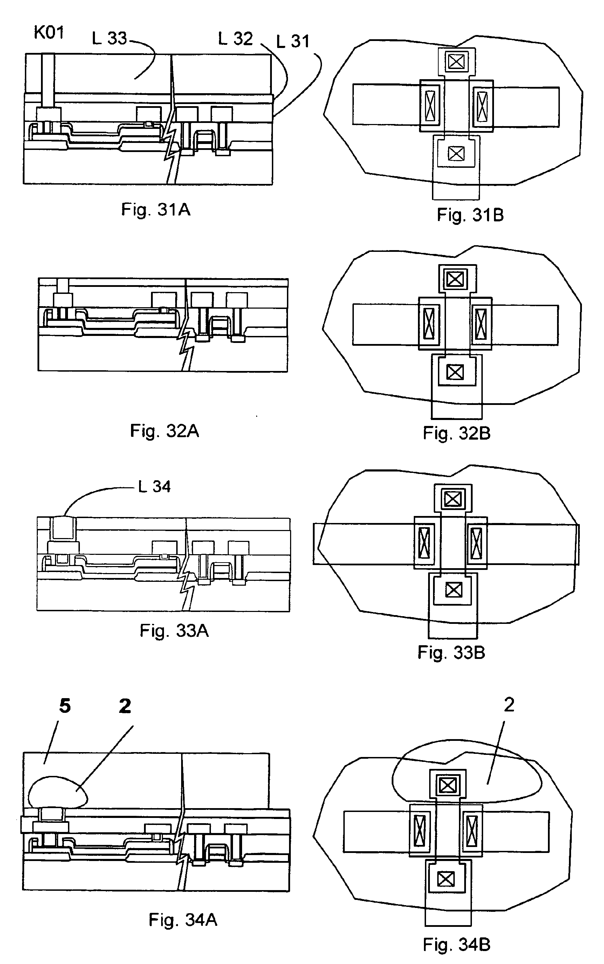

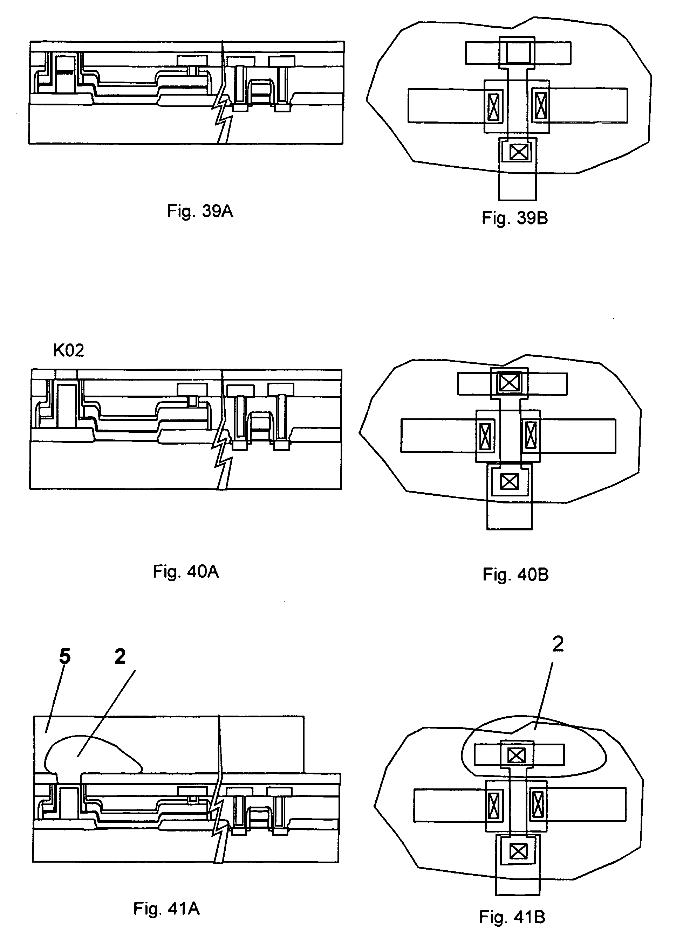

In den Zeichnungen zeigt Fig. 1 eine schematische Darstellung einer erfindungsgemäßen Kopplungsanordnung mit einem Doppelgatetransistor als elektrischen Kopplungselement. Fig. 2 zeigt die Ausführungsform von Fig. 1 zusammen mit dem elektrischen Ersatzschaltbild einer angekoppelten Zelle. Fig. 3 zeigt die Ausführungsform der erfindungsgemäßen Kopplungsanordnung von Fig. 1 mit einem vorgeschalteten Auswahltransistor. Die Figuren 4 und 5 zeigen Matrizenanordnungen der erfindungsgemäßen Kopplungsanordnung. Fig. 6 zeigt die die Ankopplung mehrerer Zellen, die sich in einem Behältnis mit Nährlösung befinden, an erfindungsgemäße Kopplungsanordnungen. Die Figuren 7 bis 33 zeigen ein Verfahren zur Herstellung einer erfindungsgemäßen Kopplungsanordnung mit einem Doppelgatetransistor als elektrischem Kopplungselement. In den Figuren 34 bis 41 sind verschiedene Varianten der erfindungsgemäßen Kopplungsanordnung mit Doppelgatetransistor dargestellt.

In den folgenden Ausführungen werden gleiche oder ähnliche Elemente mit denselben Bezugszeichen bezeichnet, so dass eine wiederholte Beschreibung dieser Elemente entfallen kann.

Fig. 1 zeigt eine schematische Darstellung einer erfindungsgemäßen Anordnung zur elektrischen Ankopplung einer biologischen Zelle 2 an diese Anordnung.

Die lebende Zelle 2 befindet sich in einer Nährlösung 5, die einen Elektrolyten darstellt. Die Nährlösung 5 ist in einem Nährlösungs-Behältnis 3 angeordnet. Das Potential der Nährlösung 5 wird über eine Referenzelektrode 4, z. B. eine Platinelektrode oder eine Wasserstoffelektrode, auf einem definierten Potential, z.B. Massepotential oder Potential Uq gegenüber Masse, festgehalten.

Die elektrische Ankopplung der Zelle 2 an einen elektronischen Schaltkreis erfolgt durch einen direkten galvanischen Kontakt mit einem Kontaktelement 1 aus einem leitfähigen Material, z. B. einem Metall, das eine geringe oder eine vernachlässigbare biologische Wirkung hat, wie z. B. Platin, Iridium, Osmium oder Gold, oder einem dotierten mono- oder polykristallinem Halbleiter, wie z.B. leitfähiges Polysilizium, oder einem leitenden Kunststoff.

Das Kontaktelement 1 ist an Punkt P mit dem Gateanschluss eines Gates FG eines Transistors T1 verbunden, der über ein zweites Gate CG verfügt. Demgemäß wird der Transistor T1 als Doppelgatetransistor bezeichnet. Das erste Gate FG des Transistors ist das innere Gate. Das zweite bzw. äußere Gate CG wird auch als Steuer- oder Kontrollgate bezeichnet. Die Bezeichnung erstes bzw. zweites Gate ergibt sich aus der zeitlichen Abfolge bei der Herstellung eines typischen Doppelgatetransistors. Source- und Drainanschlüsse des Doppelgatetransistors T1 sind mit A bzw. B bezeichnet.

Ohne dass dies als Einschränkung des allgemeinen Ansatzes zu interpretieren ist, ist in den Figuren das Referenzpotential als Massepotential M dargestellt. Selbstverständlich kann jedoch auch jedes andere Potential bzw. eine Referenzspannung dafür verwendet werden, ohne dass die Funktion der Anordnung beeinträchtigt wird.

Im Folgenden wird das Funktionsprinzip der erfindungsgemäßen Anordnung beschrieben.

Jedes Membranpotential und jede Änderung des Membranpotentials der Zelle 2 oder des auf dem Kontaktbereich des Kontaktelements 1 liegenden Zellteiles wird über das Kontaktelement 1 auf das erste Gate FG des Doppelgatetransistors T1 übertragen und dieses erste Gate FG entsprechend aufgeladen. Die Aufladung des ersten Gates FG wirkt sich auf die Ausbildung der Ladung im Kanalbereich zwischen Source und Drain des Transistors T1 aus und bewirkt auf diese Weise eine Änderung des Leitwertes bzw. der Einsatzspannung. Diese Änderung der Kanaleigenschaften kann nun, je nach Einsatzbedingung, bei festgehaltener Spannung über den Strom, und bei festem Strom über den Spannungabfall detektiert, bewertet und weiterverarbeitet werden.

Da das erste Gate FG innerhalb des elektronischen Teiles der erfindungsgemäßen Kopplungsanordnung vollständig isoliert ist, bleibt die Ladung darauf bis auf geringfügige Leckstromverluste bestehen, und der Zustand der Aufladung kann über die Wirkung auf die Einsatzspannung bzw. der Leitfähigkeit des Kanals des Transistors T1 beliebig oft überprüft, d. h. ausgelesen werden.

Allgemein gesprochen stellt diese elektronische Kopplungsanordnung damit eine Art nichtflüchtige Speicherzelle dar, die durch die Aktionspotentiale der Zelle 2 aufgeladen d.h. "beschrieben" wird. Der Ladungszustand bildet daher die Aktionspotentiale der Zellen ab. Die Auswertung der davon abhängigen Leitwerte, Ströme oder Spannungen liefert ein entsprechendes Signal im Ausgangskreis, das über einen Auswahltransistor T2 abgerufen werden kann (siehe Fig. 3). Dieses Signal stellt in unserem Kontext das "Lesen" der Aktionspotentiale dar.

Dieses Lesen erfolgt wegen der vollständigen Isolation des Ausgangskreises vom Eingangskreis ohne Veränderung der Eingangsgröße, d.h. ohne jede wesentliche Rückwirkung auf die Zelle 2.

In Fig. 1 ist ein prinzipieller Aufbau der erfindungsgemäßen Kopplungsanordnung dargestellt. Die lebende Zelle 2 befindet sich in einem Behältnis 3 aus Nährlösung 5, auf dessen Boden die Kontaktstelle eines Kontaktelements 1 schematisch dargestellt ist. Dieses Kontaktelement 2 ist an Punkt P mit dem Gate FG des Doppelgatetransistors T1 verbunden. Die Zelle 2 als Trägerin und Erzeugerin des elektrischen Signals kann als Quelle bzw. Generator Sz von Signalpulsen aufgefasst werden, wie im elektrischen Ersatzschaltbild EZ der Zelle in Fig. 2 dargestellt. Aus dem Ersatzschaltbild EZ ist auch ein Innenwiderstand Rz der Zelle ersichtlich. Im Sinne der Erfindung können die Kontakstellen der Kontaktelemente 1 prinzipiell an allen, von der Nährlösung 5 erreichbaren, benetzten, bzw. umspülten Grenzflächen der Innenwand des Behältnisses 3 angebracht werden. Die Quelle Uq ist optional und soll darstellen, dass das Elektrolytpotential der Nährlösung 5 auch verschieden vom Massepotential M bzw. vom Referenzpotential sein kann. Die erfindungsgemäße Kopplungsanordnung gemäß Fig. 2 bildet eine Ausleseschaltung. Die biologische Zelle wirkt dabei als Signalquelle Sz, die das erste Gate FG des Doppelgatetransistors T1 auflädt.

In Fig. 3 ist eine erfindungsgemäße Möglichkeit zur Auslesung von Zellsignalen aus der Kopplungsschaltung von Fig. 2 schematisch dargestellt. Die Schaltung von Fig. 3 eignet sich besonders zur Auslesung von Zellsignalen bei matrizenförmiger Anordnung der erfindungsgemäßen Kopplungsschaltung mit Doppelgatetransistor T1. Dazu ist dem Doppelgatetransistor T1 vor dem Anschluss A ein Auswahltransistor T2 vorgeschaltet (zwischen den Punkten A1 und A), der über ein Auswahlgate AG angesteuert wird. Die Funktionsweise der Schaltung ist folgende: Liegt zwischen den Punkten A 1 und B eine Spannung an, so fließt in diesem Zweig so lange kein Strom, solange der Auswahltransistor T2 sperrt. Öffnet der Auswahltransistor T2, d. h. wird er durch Anlegen eines entsprechenden Potentials am Auswahlgate AG geöffnet, so hängt der im Zweig A-B fließende Strom I von der Spannung am Anschluss C des Steuergates CG des Doppelgatetransistors T1 sowie von der Ladung am ersten Gate FG dieses Transistors ab, die wiederum vom "Signalgenerator" Sz, d.h. dem Signalzustand der biologischen Zelle abhängt. Schaltet das Steuergate CG den Transistor T1 ein, dann hängt der Strom I durch den Transistor allein vom Ladungszustand des ersten Gates FG ab. Der Strom I liefert also ein Maß für die Aufladung des ersten Gates FG, mithin für den Signalzustand der biologischen Zelle.

In den Figuren 4 und 5 ist eine Anordnung der erfindungsgemäßen Kopplungsschaltung in Form einer Matrix dargestellt. Dabei sind Spalten-Adressleitungen j, j+1 mit den Anschlusspunkten A eines jeweiligen Doppelgatetransistors T1 verbunden, wobei noch - wie in Fig. 3 gezeigt - ein nicht dargestellter Auswahltransistor dazwischengeschaltet sein kann. Zeilen-Adressleitungen i, i+1 sind mit dem Anschluss C des Steuergates CG verbunden (bzw. können mit dem Auswahlgate des Auswahltransistors verbunden sein). Die biologischen Zellen stellen einen Generator Sz für elektrische Pulse dar.

Zur Darstellung der Robustheit und Fehlertoleranz der erfindungsgemäßen Kopplungsanordnung ist in Fig. 6 der ganz allgemeine Fall einer Vielzahl erfindungsgemäßer Kopplungsanordnungen bei Vorhandensein einer größeren Anzahl von Zellen mit Fehlbelegung und Zellausfall dargestellt. Es ist leicht ersichtlich, dass das Fehlen einer Zelle (bei 0) auf einem Kontaktelement 1 über den Doppelgatetransistor T1 leicht erkannt werden kann. Solange der Doppelgatetransistor T1 (bzw. ein nicht dargestellter vorgeschalteter Auswahltransistor) ausgeschaltet bleibt, ist dieses Kontaktelement 1 an der Stelle 0 unwirksam. Ebenso kann eine dysfunktionale Zelle (2C) dadurch erkannt werden, dass sie keine oder eine unzureichende Art von Reiz/Reaktions-Muster zeigt. Auch diese Zelle 2C kann mittels des zugehörigen Doppelgatetransistors T1 problemlos ausgeschaltet werden. Die Zellen 2B, 2D und 2E zeigen ein zufriedenstellendes Reiz/Reaktionsmuster, d.h. die Zellen reagieren aktiv/passiv und weisen somit voll funktionierenden Zellkontakt auf.

Zusammengefasst kann festgehalten werden, dass die Anordnung über eine entsprechende Addressierschaltung und Adressiervorgänge, d.h. Algorithmen, erlaubt, Fehlkontakte (0) und dysfunktionale Zellen (2C) von der weiteren Interaktion auszuschließen, ohne dass es zu einer Beeinträchtigung der Interaktion der restlichen Zellen (2B, 2D, 2E) kommt.

Bisher nicht besprochen wurde die Möglichkeit einer Reizung der biologischen Zelle. Diese Möglichkeit ergibt sich durch eine spezielle Beschaltung, die dergestalt augeführt wird, dass Ladungsträger von außerhalb, d.h. z.B. vom Bulkbereich des Doppelgatetransistors T1 entweder über so genanntes Tunneln, z. B. Fowler-Nordheim-Tunneln, auf das erste Gate FG aufgebracht werden und dieses damit aufladen, oder durch Injektion von so genannten heißen Ladungsträgern aus dem Kanalbereich, oder aber durch Injektion von Ladungsträgern aus dem Steuergate CG, mit jeweils der gleichen Wirkung einer Aufladung des ersten Gates FG und damit der Reizung der Zelle mit einem elektrischen Signal.

Ohne Einschränkungen kann im Sinne der erfindungsgemäßen Anordnung der Behälter mit der Nährlösung und den Zellen auch direkt auf einem Halbleiterchip, der die gesamte oder Teile der Elektronik enthält, aufgebracht werden. In diesem Falle kann die oberste Schicht, die so genannte Passivierung, so ausgeführt werden, dass die Kontakte an speziellen Durchstoßungspunkten angebracht werden und diese dann mit den Zellen in Kontakt gebracht werden.

Ebenso können Bauelemente mit diesen Kontakten an der Oberfläche direkt in ein lebendes Gewebe eingebracht werden. Für den Kontakt mit der Gewebeflüssigkeit, dem Elektrolyten, muss dann ein geeigneter, gegebenenfalls auch integrierter Kontakt vorgesehen werden.

Im Folgenden wird ein Ausführungsbeispiel der erfindungsgemäßen Kopplungsanordnung mit einem Doppelgatetransistor beschrieben.

Die einzelnen Verfahrensschritte sind in Fig. 7A bis 33A im Querschnitt und in Fig. 7B bis 33B als sogenanntes Layout zu sehen, wobei im nachfolgenden Text Figurennummern ohne die Indizes A oder B gemeinsame Bezugnahmen auf Querschnitt und Layout sind.

Auf einem Substrat (Layer 10) (siehe Fig. 7), das im allgemeinen p-dotiertes Silizium sein wird, werden drei Schichten hintereinander abgeschieden bzw. erzeugt (Fig. 8), Layer 11 (z.B. Silizium-Dioxid), Layer 12 (z.B. Poly-Silizium), Layer 13 (z.B. Silizium-Nitrid).

Anschließend wird mit Hilfe einer Fototechnik 1 über eine Lackmaske (Layer 14) der Transistorbereich T0 definiert (Fig. 9) und der so definierte Bereich durch Ätzung von Layer 13 erzeugt (Fig. 10).

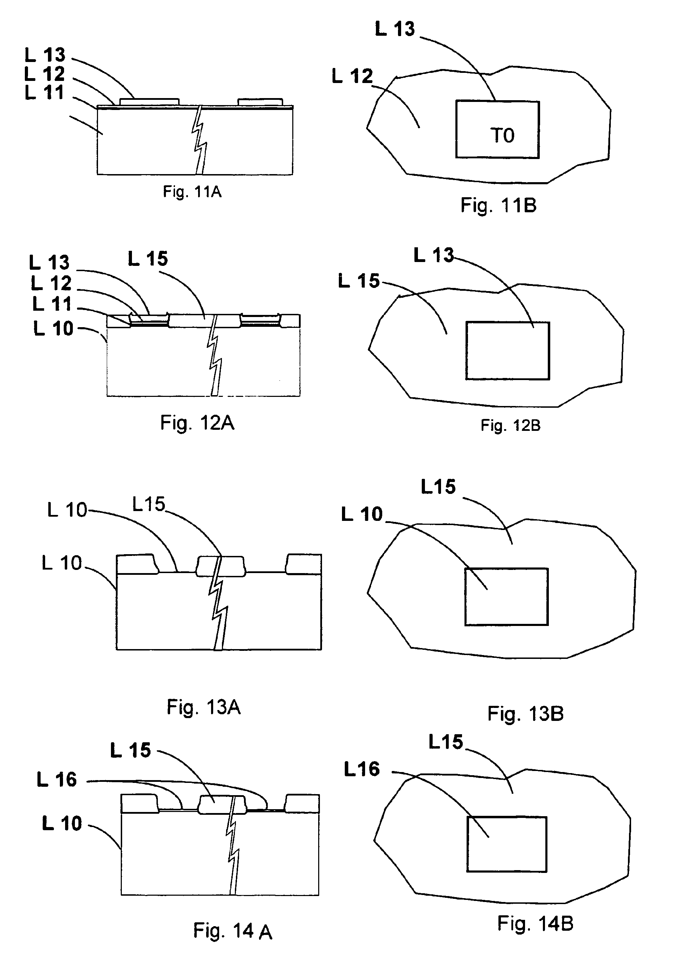

Als nächster Schritt wird die Lackmaske entfernt (Fig. 11) und der außerhalb von T0 liegende Bereich oxidiert (LOCOS, Lokale Oxidation von Silizium), um außerhalb des Transistorbereiches T0 den so genannten Feldoxidbereich (Layer 15) zu erzeugen (Fig. 12). Der Feldoxidbereich kann jedoch auch durch jede andere Technik, z.B. Shallow Trench Isolation erzeugt werden.

Nach der Oxidation werden die verbliebenen Teile der Schicht 13, 12, 11 entfernt (Fig. 13). Gegebenenfalls nach einem geeigneten Überätzungsschritt zur Entfernung des Restoxides (z.B. natives Oxid) wird der Transistorbereich freigelegt und der Gatestack aufgebaut: Hierzu wird nach Wachsen des ersten Gate-Oxides (Layer 16) (Fig. 14) oder eines anderen geeignete Dielektrikums eine erste Poly-Silizium-Schicht (Layer 17) (Fig. 15) entweder dotiert abgeschieden, oder undotiert abgeschieden und nachträglich dotiert (z.B. n-dotiert). Anschließend wird auf diese Poly-Silizium-Schicht eine dünne Silizium-Dioxid-Schicht oder ein anderes geeignetes Dielektrium aufgewachsen (Layer 18) oder abgeschieden, und dann wird eine weitere, die so genannte zweite Poly-Silizium-Schicht dotiert abgeschieden oder undotiert abgeschieden und nachträglich dotiert (Layer 19) (Fig. 16).

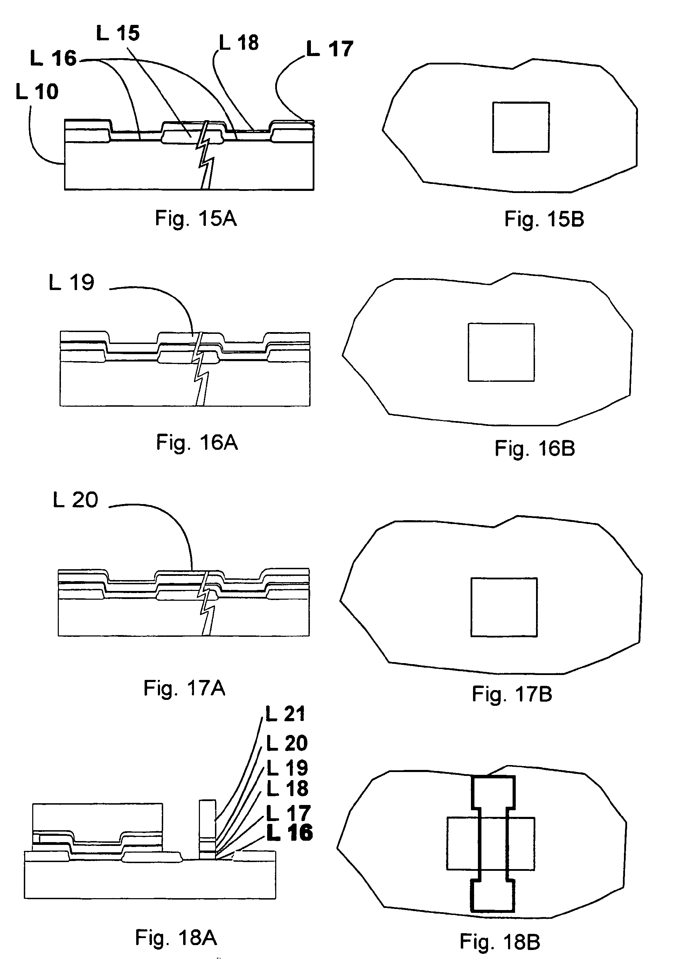

Auf diese Schicht wird eine weitere dielektrische Schicht (Layer 20) abgeschieden oder aufgewachsen (z.B. Silizium-Dioxid) (Fig. 17). Im nächsten Schritt (Fig. 18) wird photolithographisch (Layer 21) das Gate G0, bzw. die Gate-Ebene definiert und anschließend durch Strukturierung der Layers 20, 19, 18, 17 und 16 erzeugt. Bei diesem Vorgang werden das erste Gate G1 (Layer 17) und das zweite Gate G2 (Layer 19) erzeugt.

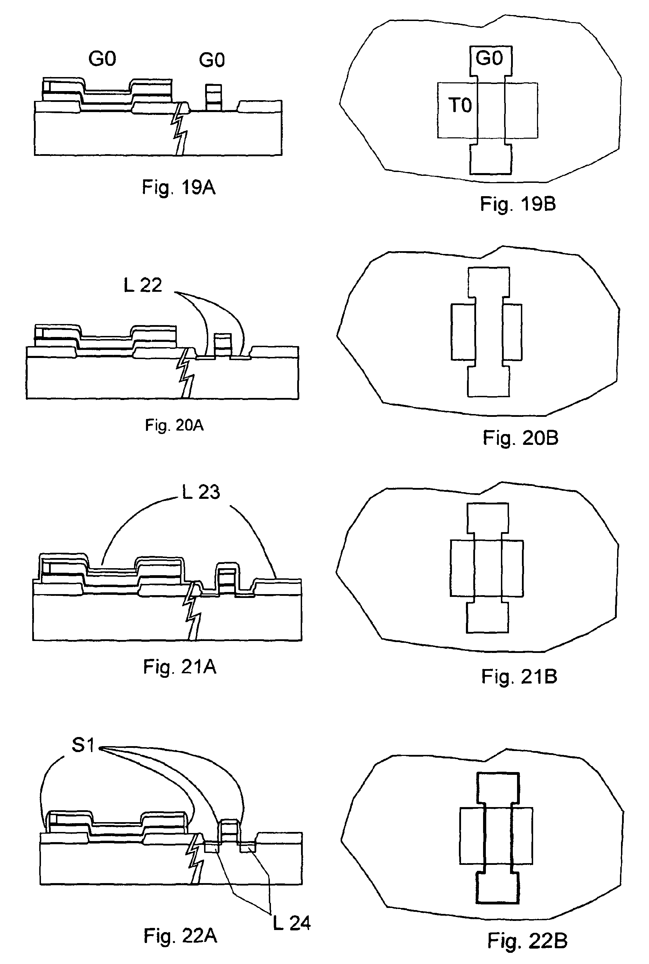

Dann wird der Fotolack (Layer 21) entfernt (Fig. 19) und über einen Implantationsvorgang, oder über einen anderen geeigneten Dotierprozess, wird die so genannte Lightly Doped Drain (LLD-)Zone (Layer 22) Zone selbstjustiert erzeugt (Fig. 20). Im nächsten Schritt wird durch konforme Abscheidung eines Dielektrikums (Layer 23) (Fig. 21) und anschließende anisotrope Rückätzung ein so genannter Seitenwand-Spacer (Layer 24) erzeugt (Fig. 22).

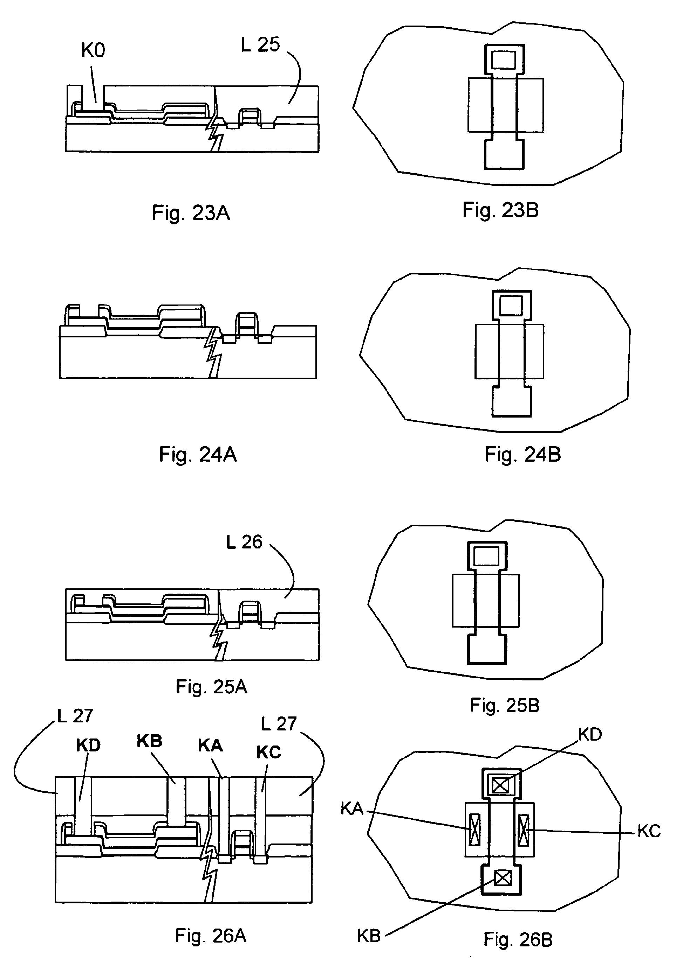

Nach der Erzeugung des Spacers S 1 wird die so genannte Heavily Doped Drain (HDD) Implantation durchgeführt (Layer 25) (Fig. 23) und der Source/Drain Anschlussbereich definiert (Fig. 24).

Im dargestellten Ausführungsbeispiel wird im Anschluss an diese HDD-Implantation mittels einer Fototechnik 3 das Kontaktfenster (K0) definiert (Layer 26) (Fig. 25) und durch Ätzung der Layers 20, 19 und gegebenenfalls 18 erzeugt. Nach Entfernen des Lacks (Layer 26) wird eine dicke dielektrische Einfach- oder Mehrfachschicht (Fig. 26) abgeschieden und gegebenenfalls planarisiert oder planarisiert abgeschieden (Layer 27).

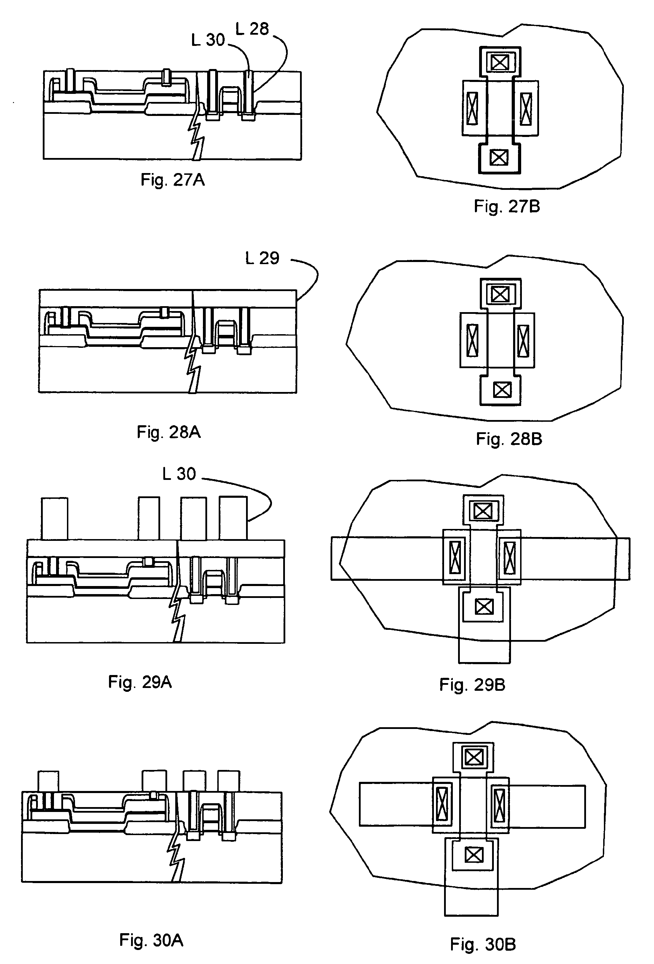

Anschließend werden über eine Fototechnik 4 die Kontaktlöcher KA, KB, KC, KD definiert (Layer 28) (Fig. 27) und durch Ätzung der entsprechenden Schichten Öffnungen erzeugt, die zu Source, Drain, Gate1 und Gate 2 reichen.

Die Kontaktlöcher werden gegebenenfalls unter Zuhilfenahme einer Barriereschicht (Layer 29) (Fig. 28) mit einem leitenden Material (z.B. Wolfram / Titannitrid) aufgefüllt (Layer 30) (Fig. 29 u. 30).

Im nächsten Schritt wird eine metallische Schicht (Layer 31) aufgebracht (Fig. 31) und mit einer Fototechnik 5 strukturiert. Nach Fertigstellung der Metallisierungsebene M1 (Layer 31) werden im Ausführungsbeispiel eine, zwei oder mehrere Dielektrikschichten aufgebracht, die entweder planarisiert sind oder planarisiert werden (in Fig. 31 zwei Schichten, Layer 32 und Layer 33).

Nach Abscheidung dieser Schichten, deren Aufgabe im wesentlichen darin besteht, den Transistorbereich inklusive Metallisierung von der Außenwelt chemisch und elektrisch zu isolieren (passivieren), wird über eine Fotolithographie 6 (Layer 33) ein Kontaktloch K01 zum Gate 1 definiert und durch Ätzung erzeugt (Fig. 31 und 32). Anschießend wird der fotolithographische Lack (Layer 34) entfernt und das verbliebene Kontaktloch K01 wird mit einer Barriere/Haftschicht (Layer 35) und einem leitenden Material (Layer 36) aufgefüllt (Fig. 33) und zwar so, dass die Oberfläche im wesentlichen plan bleibt. Dieser Kontaktbereich stellt die erfindungsgemäße Ankoppelung an eine biologische Zelle her (Fig. 34A (Querschnitt) und 34B (Layout)). Wie aus Fig. 34 ersichtlich ist, kann der Zellkontakt direkt über diese Andockstelle hergestellt werden. Die hier beschriebene Abbildung sei Variante A genannt.

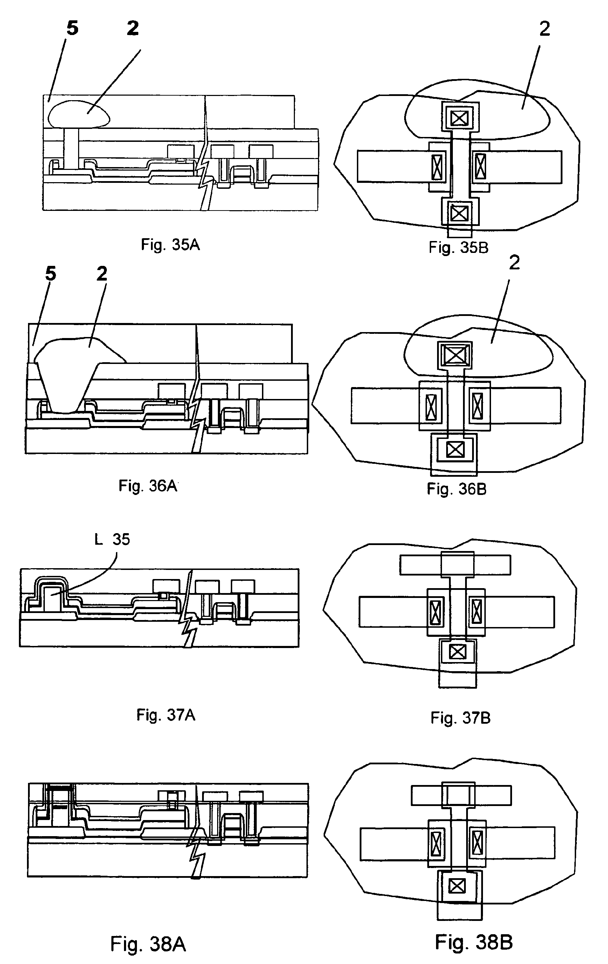

In einer Variante B kann ohne Einschränkung der Erfindung dieser Kontakt, wie in Fig. 35A, 35B gezeigt, in einem einzigen Schritt direkt bis zum Gate 2 hergestellt werden. Zu diesem Zweck muss die Kontaktlochätzung nach Abschluss der Herstellung des integrierten Schaltkreises durchgeführt werden. Der Einfacheit halber ist eine eventuell vorhandene Haft- bzw. Barriereschicht nicht extra eingezeichnet.

In einer Variante C der Erfindung kann der Zellkontakt auch ohne Inanspruchnahme eines metallischen Zwischenstückes erfolgen, wie in Fig. 36 dargestellt.

In einer Variante D, die in den Figuren 38A bis 41A im Querschnitt und in Figuren 38B bis 41B im Layout dargestellt ist, kann durch Einführung einer sogenannten Supportebene (Herstellvorgang siehe Fig. 37), die im Kern eine Schwelle erzeugt (Layer 37), das Gate 1, oder eine Zuleitung dazu, direkt an die Oberfläche geführt werden. Dabei wird nur die Komformität der Poly-Silizium-Abscheidung und der Dielektrika ausgenutzt.

Für die Kontaktherstellung ist wie in der Varianten B, C und D ein Lithographie-Schritt notwendig, der erlaubt, einen Kontakt K02 zu Gate 1 bzw. zu dessen Zuleitung herzustellen. Die eigentliche Kontaktierung mit der Zelle erfolgt dann wie in Variante C. Im Sinne der Erfindung kann jedoch auch eine Kontaktlochauffüllung eingesetzt werden.

## Patentansprüche

1. Anordnung zur Kopplung einer lebenden Zelle (2), insbesondere Nervenzelle, an eine elektronische Schaltung zur Aufnahme von direkt oder indirekt elektrisch wirksamen Zellsignalen und/oder zur elektrischen Reizung der Zelle (2), wobei die Kopplungsanordnung einen Transistor (T1) mit einem doppelt ausgeführten Gate umfasst, sowie eines der Gates als Steuergate (CG) zur Ansteuerung des Transistors über externe Steuersignale ausgebildet ist, während das andere der Gates (FG) mit einem elektrisch leitenden Kontaktelement (1) verbunden ist, das mit der Zelle (2) zur Erfassung von Änderungen der elektrischen Eigenschaften der Zelle in Berührung bringbar ist.

2. Kopplungsanordnung nach Anspruch 1, **dadurch gekennzeichnet, dass** ein Nährlösungs-Behältnis (3) vorgesehen ist, in dessen Innenraum zumindest ein Kontaktelement (1) ragt oder diesen zumindest teilweise bildet.

3. Kopplunganordnung nach Anspruch 2, **dadurch gekennzeichnet, dass** eine mit einer Referenzspannung (Uq) verbundene elektrisch leitende Referenzelektrode (4) vorgesehen ist, die in den Innenraum des Nährlösungs-Behältnisses (3) ragt.

4. Kopplungsanordnung nach einem der Ansprüche 1 bis 3, **dadurch gekennzeichnet, dass** das elektrisch leitende Kontaktelement (1) ein Material mit geringer biologischer Wirkung umfasst, vorzugsweise ausgewählt aus hochschmelzenden Metallen, wie Platin, Iridium, Osmium, Wolfram oder Gold, oder Legierungen daraus; oder aus Halbleitersiliziden, wie Platinsilizid, Wolframsilizid, Titansilizid; oder aus einem dotierten monokristallinen oder polykristallinen Halbleiter, wie leitfähigem Polysilizium; oder aus leitfähigen Kunststoffen.

5. Kopplungsanordnung nach einem der vorhergehenden Ansprüche, wobei eine Vielzahl von Kopplungsanordnungen in Form einer Matrix (10) mit Zeilen (i, i+1)und Spalten (j, j+1)angeordnet ist, **dadurch gekennzeichnet, dass** jede Kopplungsanordnung durch eine Adressierschaltung einzeln oder gruppenweise über entsprechende Aktivierung von Spalten- und Zeilen-Adressleitungen ansteuerbar ist.

6. Kopplungsanordnung nach Anspruch 5, **dadurch gekennzeichnet, dass** jede Kopplungsanordnung einen Auswahlschalter mit einem Steueranschluss, insbesondere einen Auswahltransistor (T2) mit einem Auswahl-Gate (AG) umfasst, wobei ein Eingang (A1) des Auswahlschalters mit einer Zeilen-Adressleitung verbunden ist und der Steueranschluss (AG) des Auswahlschalters mit einer Spalten-Adressleitung verbunden ist.

7. Kopplungsanordnung nach Anspruch 6, **dadurch gekennzeichnet, dass** die Adressierschaltung Adressierauswertemittel zur Erkennung von disfunktionalen Zellen und von Fehlkontakten zwischen Kontaktelement (1) und Zelle (2) aufweist, wobei bei einer solchen Erkennung gegebenenfalls die weitere Interaktion dieser Zellen bzw. Kontaktelemente selektiv unterbrechbar ist.

8. Kopplungsanordnung nach Anspruch 7, **dadurch gekennzeichnet, dass** zur selektiven Unterbrechung der Interaktion von Zellen Unterbrechungsmittel, wie ein elektronischer Schalter (T2), vorgesehen sind.

9. Kopplungsanordnung nach einem der vorhergehenden Ansprüche, **dadurch gekennzeichnet, dass** eine Vielzahl von Kopplungsanordnungen auf einem Chip angeordnet ist, wobei der Chip vorzugsweise in Si-Planartechnologie hergestellt und gegebenenfalls mit anderen Technologien, wie Schaltungen für lokale Verstärkung, "on-chip-logic", oder "systems on chips (SoC)" integriert ist.

10. Kopplungsanordnung nach Anspruch 9 in Verbindung mit Anspruch 2, **dadurch gekennzeichnet, dass** das Nährlösungs-Behältnis (3) auf dem Chip angeordnet ist.

11. Kopplungsanordnung nach einem der Ansprüche 1 bis 10, **dadurch gekennzeichnet, dass** das mit dem elektrisch leitenden Kontaktelement (1) verbundene Gate (FG) des Doppelgatetransistors (T1) mit externen Ladungsträgern aufladbar ist.

12. Kopplungsanordnung nach Anspruch 11, **dadurch gekennzeichnet, dass** zur Aufladung des mit dem elektrisch leitenden Kontaktelement (1) verbundenen Gate (FG) mit externen Ladungsträgern Tunnelbildung, z.B. Fowler-Nordheim-Tunneln, zwischen dem Gate und dem Bulkbereich des Doppelgatetransistors, oder Injektion von so genannten heißen Ladungsträgern aus dem Kanalbereicht des Doppelgatetransistors (T1), oder Injektion von Ladungsträgern aus dem Steuergate (CG) des Doppelgatetransistors (T1) vorgesehen ist.

## Claims

1. An array to couple a live cell (2), in particular a nerve cell, to an electronic circuit to pick up directly or indirectly electrically effective cell signals and/or to electrically stimulate the cell (2), wherein the coupling array comprises a transistor (T1) with a double-designed gate and one of the gates is designed as a control gate (CG) to select the transistor via external control signals, whereas the other one of the gates (FG) is connected to an electrically conducting contact element (1) which can be attached to the cell (2) in order to register changes in the electric properties of the cell.

2. A coupling array according to claim 1, **characterized in that** a container (3) for a nutrient solution is provided, with at least one contact element (1) projecting into the interior thereof or at least partly Forming said interior.

3. A coupling array according to claim 2, **characterized in that** an electrically conducting reference electrode (4) connected to a reference voltage (Uq) is provided, which reference electrode projects into the interior of the container (3) for the nutrient solution.

4. A coupling array according to any of claims 1 to 3, **characterized in that** the electrically conducting contact element (1) comprises a material of low biological effect, preferably selected from refractory metals such as platinum, iridium, osmium, tungsten or gold, or alloys thereof; or from semiconductor silicides such as platinum silicide, tungsten silicide, titanium silicide; or from a doped monocrystalline or polycrystalline semiconductor such as conductive polysilicon; or from conductive plastics.

5. A coupling array according to any of the preceding claims, wherein a large number of coupling arrays are arrayed in the form of a matrix (10) of lines (i, i+1) and columns (j, j+1), **characterized in that** each coupling array can be selected by an address circuit singly or in groups by appropriately activating column and line address circuits.

6. A coupling array according to claim 5, **characterized in that** each coupling array comprises a selection switch with a control connection, in particular a selection transistor (T2) with a selection gate (AG), with an input (A1) of the selection switch being connected to a line address circuit and the control connection (AG) of the selection switch being connected to a column address circuit.

7. A coupling array according to claim 6, **characterized in that** the address circuit has address evaluation tools to detect dysfunctional cells and faulty contacts between the contact element (1) and the cell (2), where, in the case of such detection, further interaction between these cells and contact elements, respectively, can optionally be selectively interrupted.

8. A coupling array according to claim 7, **characterized in that** interrupting tools such as an electronic switch (T2) are provided for the selective interruption of interaction between cells.

9. A coupling array according to any of the preceding claims, **characterized in that** a large number of coupling arrays are arrayed on a chip, the chip preferably being produced according to the Si-planar technology and optionally being integrated with other technologies such as circuits for local amplification, on-chip logic or systems on chips (SoC).

10. A coupling array according to claim 9 in connection with claim 2, **characterized in that** the container (3) for the nutrient solution is placed on the chip.

11. A coupling array according to any of claims 1 to 10, **characterized in that** the gate (FG) of the double-gate transistor (T1), which gate is connected to the electrically conducting contact element (1), can be charged with external charge carriers.

12. A coupling array according to claim 11, **characterized in that**, for charging the gate (FG) connected to the electrically conducting contact element (1) with external charge carriers, tunnelling is provided, e.g. Fowler Nordheim tunnels, between the gate and the bulk area of the double-gate transistor, or injection of so-called hot charge carriers from the channel area of the double-gate transistor (T1), or injection of charge carriers from the control gate (CG) of the double-gate transistor (T1).

## Revendications

1. Dispositif pour coupler une cellule vivante (2), en particulier une cellule nerveuse, à un circuit électronique pour l'enregistrement de signaux cellulaires à effet électrique direct ou indirect et/ou pour l'excitation électrique de la cellule (2), le dispositif de couplage comprenant un transistor (T1) avec une porte exécutée en double, et aussi l'une des portes est formée en tant que porte de commande (CG) pour commander le transistor par l'intermédiaire de signaux de commande externes, tandis que l'autre porte (FG) est reliée à un élément de contact conduisant l'électricité (1), qui peut être amené en contact avec la cellule (2) pour capter des variations des propriétés électriques de la cellule.

2. Dispositif de couplage selon la revendication 1, **caractérisé en ce qu'**un récipient de solution nutritive (3) est prévu, dans l'espace intérieur duquel au moins un élément de contact (1) se projette ou forme celui-ci au moins partiellement.

3. Dispositif de couplage selon la revendication 2, **caractérisé en ce qu'**une électrode de référence (4) conduisant l'électricité, reliée à une tension de référence (Uq), est prévue, qui se projette dans l'espace intérieur du récipient de solution nutritive (3).

4. Dispositif de couplage selon l'une quelconque des revendications 1 à 3, **caractérisé en ce que** l'élément de contact (1) conduisant l'électricité comprend un matériau ayant un effet biologique réduit, de préférence choisi parmi les métaux à point de fusion élevé, comme le platine, l'iridium, l'osmium, le tungstène ou l'or, ou leurs alliages ; ou parmi les siliciures de semi-conducteurs comme le siliciure de platine, le siliciure de tungstène, le siliciure de titane ; ou parmi un semi-conducteur monocristallin ou polycristallin, dopé, comme le polysilicium conducteur ; ou parmi les matières plastiques conductrices.

5. Dispositif de couplage selon l'une quelconque des revendications précédentes, dans lequel une pluralité de dispositifs de couplage est disposée sous forme d'une matrice (10) avec des lignes (i, i+1) et des colonnes (j, j+1), **caractérisé en ce que** chaque dispositif de couplage peut être commandé individuellement par un circuit d'adressage ou par groupe par l'intermédiaire d'une activation correspondante de lignes d'adressage de colonne et de ligne.

6. Dispositif de couplage selon la revendication 5, **caractérisé en ce que** chaque dispositif de couplage comprend un commutateur de sélection ayant une connexion de commande, en particulier un transistor de sélection (T2) avec une porte de sélection (AG), une entrée (A1) du commutateur de sélection étant reliée à une ligne d'adressage de ligne, et la connexion de commande (AG) du commutateur de sélection étant reliée à une ligne d'adressage de colonne.

7. Dispositif de couplage selon la revendication 6, **caractérisé en ce que** le circuit d'adressage présente des moyens d'évaluation d'adresse pour reconnaître les cellules dysfonctionnelles et les contacts défectueux entre l'élément de contact (1) et la cellule (2), où dans une telle reconnaissance, l'interaction supplémentaire de ces cellules ou éléments de contact peut être interrompue sélectivement le cas échéant.

8. Dispositif de couplage selon la revendication 7, **caractérisé en ce que** des moyens d'interruption, comme un circuit électronique (T2), sont prévus pour interrompre sélectivement l'interaction de cellules.

9. Dispositif de couplage selon l'une quelconque des revendications précédentes, **caractérisé en ce qu'**une pluralité de dispositifs de couplage est disposée sur une puce, la puce étant de préférence fabriquée en Si selon une technologie planaire et intégrée éventuellement à d'autres technologies, comme des circuits pour une amplification locale, « on-chip-logic » (« logique sur puce »), ou « systems on chips » (« systèmes sur puce »).

10. Dispositif de couplage selon la revendication 9, en liaison avec la revendication 2, **caractérisé en ce que** le récipient de solution nutritive (3) est disposé sur la puce.

11. Dispositif de couplage selon l'une quelconque des revendications 1 à 10, **caractérisé en ce que** la porte (FG) reliée à l'élément de contact (1) conduisant l'électricité, du transistor à double portes (T1), peut être chargée par des porteurs de charges externes.

12. Dispositif de couplage selon la revendication 11, **caractérisé en ce que** pour charger la porte (FG) reliée à l'élément de contact (1) conduisant l'électricité avec des porteurs de charges externes, il est prévu une formation de tunnels, par exemple des tunnels Fowler-Nordheim, entre la porte et la zone interne du transistor à double porte, ou l'injection de porteurs de charges dits chauds à partir de la zone de canal du transistor à double porte (T1) ou l'injection de porteurs de charges à partir de la porte de commande (CG) du transistor à double porte (T1).
